# EUROPEAN PATENT SPECIFICATION

(11) **EP 0 824 903 B1**
(45) Date of publication and mention of the grant of the patent: **14.01.2004**
(21) Application number: 97306201.1
(22) Date of filing: 15.08.1997
(51) Int. Cl.: A61F 2/06

(54) **A profiled stent and method of manufacture**
Profilierter Stent und Herstellungsverfahren dafür
Un stent profilé et méthode de fabrication

(30) Priority: 23.08.1996 US 702258
(43) Date of publication of application: 25.02.1998
(73) Proprietor: Medtronic AVE, Inc., Santa Rosa, California 95403 (US)
(72) Inventor: Birdsall, Matthew J., Santa Rosa, California 95405 (US); Lashinski, Robert D., Sebastopol, California 95472 (US); Lashinski, Randall T., Santa Rosa, California 95403 (US)
(74) Representative: Bayliss, Geoffrey Cyril

(56) References cited:
- EP-A- 0 417 928
- WO-A-96/09020
- WO-A-96/12450
- US-A- 3 868 956
- US-A- 4 041 766
- US-A- 5 403 341

## Description

This present invention relates to intravascular stents for maintaining the patency of lumens in living tissue. And, more specifically, to a profiled stent and method of manufacture therefor.

Percutaneous transluminal coronary angioplasty ("PTCA") is a now common procedure for treating coronary artery disease. PTCA typically involves advancing a catheter, having an inflatable balloon on the distal end thereof, through a patient's arterial system until the balloon crosses an atherosclerotic lesion. The balloon is then inflated to dilate the artery. After dilation, the balloon is deflated and the catheter removed leaving an enlarged arterial passageway or lumen, thereby increasing blood flow. A significant number of PTCA procedures, however, result in a restenosis or renarrowing of the lumen.

To lessen the risk of stenosis or restenosis of lumens, various endoprosthetic devices have been proposed for mechanically keeping an affected lumen open after completion of procedures, such as PTCA. For purposes of the instant invention, a lumen can be a blood vessel, a bile duct, or any other similar body conduit that tends to improperly constrict as a result of disease or malfunction. A lumen may also be a graft (whether natural or artificial) in any type of body conduit.

Endoprosthetic devices generally referred to as stents, are typically inserted into the lumen, positioned across a lesion, and then expanded to keep the passageway clear. Effectively, the stent overcomes the natural tendency of some lumen walls to close due to restenosis, thereby maintaining a more normal flow of blood through that lumen than would be possible if the stent were not in place or if only a PTCA procedure were performed.

There are two general categories of stents, self-expanding stents and balloon-expandable stents. Some self-expanding stents are made from a tube of stainless wire braid. Such stents are typically compressed into a first shape and inserted into a sheath or cartridge. During insertion, the stent is positioned along a delivery device, such as a catheter, that is extended to make the stent diameter as small as possible. When the stent is positioned across the lesion, the sheath is withdrawn causing the stent to radially expand and abut the vessel wall. Depending on the materials used in construction of the stent, the tube maintains the new shape either through mechanical force or otherwise.

The stent is then delivered to the affected area on a catheter. Once properly positioned, the sent is allowed to expand.

Another type of self-expanding stent is made from a shape-memory alloy such as NITINOL. This stent has been pre-treated to assume an expanded state at body temperature. Prior to delivery to the affected area, the stent is typically crimped or compressed near or below at room temprature.

Balloon-expandable stents are typically introduced into a lumen on a catheter having an inflatable balloon on the distal end thereof. When the stent is at the desired location in the lumen, the balloon is inflated to circumferentially expand the stent. The balloon is then deflated and the catheter is withdrawn, leaving the circumferentially expanded stent in the lumen, usually as a permanent prosthesis for helping to hold the lumen open.

One type of balloon-expandable stent is a tubular-slotted stent, which involves what may be thought of as a cylinder having a number of slots cut in its cylindrical wall, resulting in a mesh when expanded. A tubular-slotted stent is cut out of a tube, typically a hypo-tube, or out of a sheet, which is then rolled, and then welded to form a cylinder. Tubular-slotted stents that are cut out of a tube typically have a rectangular cross-section, which produces rather sharp and square edges that remain even after polishing. As a result, such tubular-slotted stents may have a tendency to dissect the lumen as the stent is advanced through the lumen on the catheter.

A balloon-expandable stent referred to as a wire stent overcomes some of the problems associated with tubular-slotted stents. A wire stent is generally formed by winding a circular shaped wire into supportive elements, which typically have a circular cross-section. The problem with wire stents is that the supportive elements comprising the stent can axially displace with respect to each other, resulting in a stent that fails to provide adequate support.

U.S. Patent No. 5,292,331 issued to Boneau, which is hereby incorporated by reference discloses another type of wire stent, referred to here as a Boneau stent. A Boneau stent is made by taking a ring or toroid having a circular cross-section, and then forming the ring into a series of sinusoidally-shaped elements. While preferably employing a single piece of material, suitably welded wire, is also acceptable. A Boneau stent bridges the gap between tubular-slotted stents and wire stents by retaining the flexibility of wire stents, while approaching the axial stability of tubular-slotted stents.

While conventional stents have been found to work well, conventional stents suffer from several disadvantages. As stated above, stents that have a rectangular cross-section may damage the inner walls of a lumen due to sharp edges. And stents having a rounded cross-section, while reducing the risk of dissection or trauma, neither possess an efficient surface-to-wall covering ratio nor efficient strength for material volume.

Accordingly, what is needed is an improved stent structure that makes efficient use of stent material while reducing the risk of trauma to the lumen wall. The present invention addresses such a need.

WO 96/09020 discloses a stent comprising a hollow cylindrical body formed of a plurality of elongate support members which extend in a generally axial direction, at least some of the members having a cross section in a plane perpendicular to the axis of the cylinder which has a maximum dimension in the circumferential direction greater than its maximum diameter in the radial direction.

According to a first aspect of the present invention such a stent is characterised in that the cross section also has a smooth periphery and has a radially outer face which is substantially flat.

WO 96/12450 discloses a method for producing a stent to be placed in a lumen in living tissue comprising the step of: manufacturing a stent having a hollow cylindrical body formed of a plurality of elongate support members which extend in a generally axial direction;

According to a second aspect of the present invention such a method characterised by compressing at least some of the elongate support members in the radial direction so that their cross section in a plane perpendicular to the axis of the cylinder has a maximum dimension in a circumferential direction greater than its maximum diameter in the radial direction.

According to the apparatus and method disclosed herein, the present invention increases the radial strength of the stent and increases the efficiency of surface coverage. Furthermore, rounded edges are retained on the stent, which provides less traumatic trackability as the stent is advanced through a lumen.

Therefore, it is an object of the instant invention to provide a stent with increased load-carrying capability.

It is a further object of the invention to provide a stent which optimizes the stent surface to lumen wall coverage.

It is also an object of the invention to displace stent material to higher stressed regions.

These and other advantages are realized while retaining rounded edges on the stent so that it remains less traumatic.

The following is a description of some specific embodiments of the invention, reference being made to the accompanying drawings, in which:
Figure 1 is a side view of an illustrative embodiment of a stent used for forming a profiled stent embodying the principles of the present invention.
Figures 2a-2c are cross-section views of sections of various stents.
Figures 3a and 3b are cross-section views of a profiled section of a stent in accordance with the present invention.
Figure 4 is an isometric view of the Boneau stent similar to that shown in Figure 1 that has undergone the process of profiling in accordance with the present invention to produce a profiled stent.
Figure 5 is an end view of a six crown non-profiled Boneau stent.
Figure 6 is an end view of a six crown profiled Boneau stent.
Figure 7 is a flow chart depicting the process of producing a profiled stent in accordance with the present invention.
Figure 8 is a block diagram showing the profiling of a stent using a rotary swaging machine.
Figure 9 is a block diagram showing the profiling of a stent using a collet.
Figure 10 is a block diagram showing the profiling of a stent using a roller machine.

The present invention relates to a profiled stent and a method of manufacture therefor. The following description is presented to enable one of ordinary skill in the art to make and use the invention and is provided in the context of a patent application and its requirements. Various modifications to the preferred embodiment will be readily apparent to those skilled in the art and the generic principles herein may be applied to other embodiments. Thus, the present invention is not intended to be limited to the embodiment shown but is to be accorded the widest scope consistent with the principles and features described herein.

The present invention provides a profiled stent formed from a stent of conventional design. Although stents may be constructed in many different ways, the profiling method of the present invention is applicable to all known stent constructions, and it will be readily apparent from the following discussion of several exemplary constructions how the invention can be applied to any other type of stent construction.

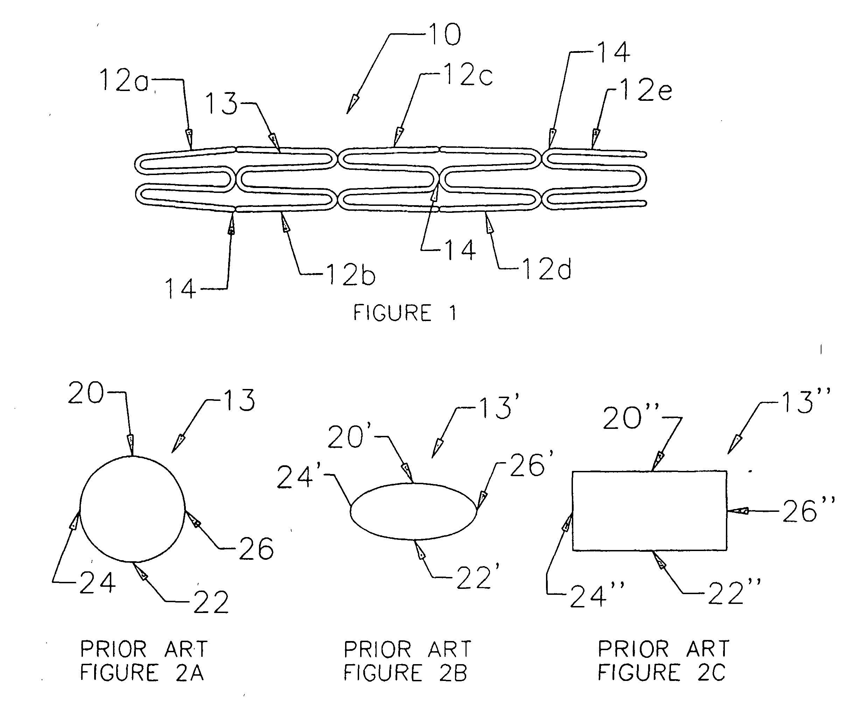

Figure 1 is a side view of an illustrative embodiment of a stent used for forming a profiled stent embodying the principles of the present invention. As described in the Boneau patent, an illustrative stent 10 includes five sections 12a-e, each of which is made of an endless metal loop that has been bent into a plurality of straight sections or struts 13 that are integrally joined by discrete axial turns, or crowns 14. Each section 12 may have more undulations than are shown in Fig. 1, but the simplified depictions shown herein will be sufficient to illustrate the present invention.

Although sections 12 may or may not be made of what would be regarded in some other arts as wire, the material of sections 12 is generally wire-like, and so the term "wire" is sometimes used herein to refer to such stent material. Axially adjacent sections 12 may be joined to one another at one or more of their crowns 14. These connections (if and to the extent present) may be made by welding, soldering, adhesive bonding, mechanical fastening, or in any other suitable manner.

A typical technique for delivering stents of the general type shown in Fig. 1 into a lumen is to initially dispose of the stent structure in a circumferentially compressed form around a deflated balloon which is part of a balloon catheter. The catheter is then inserted axially into a tubular body structure to be stented until the balloon and stent are at the desired location along the body structure. The balloon is then inflated to circumferentially expand the stent. Lastly, the balloon is deflated and the catheter is withdrawn, leaving the expanded stent behind in the body structure.

The deformation of the stent produced by the balloon as described above is at least partly permanent. As used here, such permanent deformation will be referred to as "plastic". It will be understood that the terms "plastic", "plastically", or the like as used herein mean any type of non-elastic or permanent deformation, whether in the traditional materials science sense, and therefore, due to straining some portion of the stent material beyond its elastic limit, or as a result of any other property of the stent material or structure which results in the deformed stent taking a "set", which is different from its initial set. Correspondingly, the term "yield strength" means the point at which the stent structure or its material transitions from elastic to plastic deformation, as the term "plastic" is broadly defined above.

The balloon is strong enough to overcome the yield strength of the stent, but when the balloon is no longer radially supporting the stent, the surrounding tubular body structure does not exert sufficient radially inward force on the stent to overcome the stent's yield point to the extent that the stent returns to its original diameter.

Figures 2a-2c are cross-section views of struts or support members 13 of various stent sections. Figure 2a is a cross-section view of stent 10 shown in Figure 1, which has a circular cross-sectional shape. Or as shown Figure 2b, the cross-sectional shape of strut 13' may be ellipsoidal. As stated above, stents may be constructed in many different ways. One feature common to conventional stents, however, is that they all include some type of support member or members that have substantially the same cross-sectional dimensions. Referring to Figure 2c, besides circular and ellipsoidal cross-sectional shapes, a stent section 12" may have a rectangular cross-sectional shape as shown, or a hexagonal, square, or other geometric shape.

Referring to Figures 2a-2c, the cross-sections of all types of support members may be described as generally defined by a top portion 20, 20', 20" a bottom portion 22, 22', 22" side portion 24, 24', 24", and an opposing side portion 26, 26', 26". As shown in Figures 2a-2c, once the stent is disposed and then expanded inside a lumen, the top portion 20, 20', 20" of a support member is the part that abuts against and supports the wall of the lumen. Therefore, the top and bottom portions 20, 20', 20" and 22, 22', 22" are stressed more than the side portions 24, 24', 24" and 26, 26', 26" of the support member.

Stents having traditionally shaped cross-sections as shown in Figures 2a-2c suffer from various disadvantages. One disadvantage with stents comprising support members that have a rectangular and square cross-sections is that they have sharp edges, rather than round edges, which can tear the tissue in the lumen. And, possibly, dissect the lumen.

Disadvantages associated with stents comprising support members that have a circular cross-section is that they have an inefficient surface to lumen wall coverage for the mass of material used. Nor are they optimized for radial strength. For example, a Boneau stent is collapsed along a circumferential plane by closing the crowns. [And because the material is round, it has the same strength in the circumferential plane as it would if the crown was bent in the other direction.] Referring again to Figures 2a and 2b, the side portions 24, 24', 24" and 26, 26', 26" lie along a neutral axis which does not need as much material support as the top and bottom portions 20, 20',20" and 22, 22', 22". In addition, the circular cross-section of such a support member has a large lumen wall stand-off thickness, which decreases the overall inner diameter of the stent.

According to the present invention, the cross-section of conventional stent support members are changed through a swaging technique which changes the profile of the stent such that material from the low stressed locations in the support members are moved to higher stress areas.

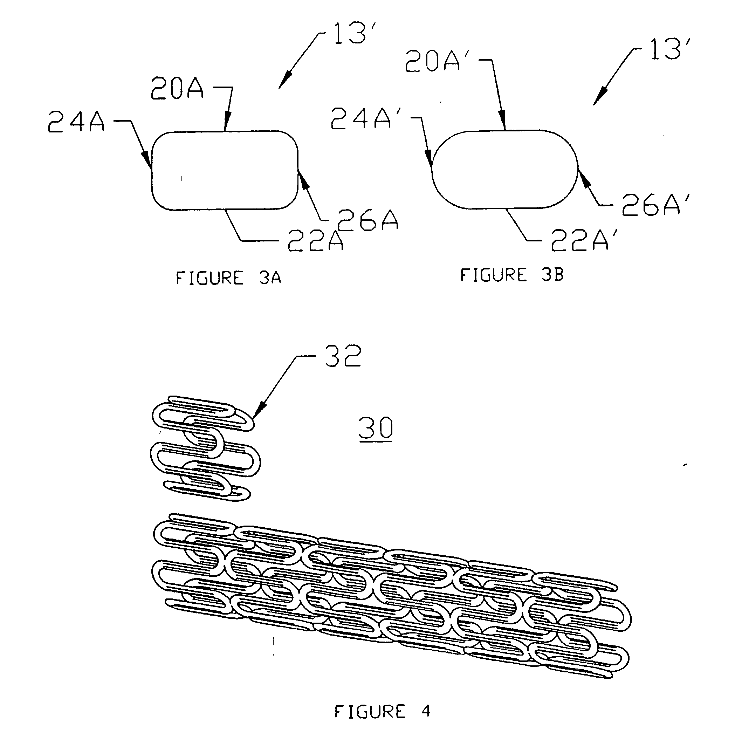

Figures 3a and 3b are cross-section views of a profiled member 13, 13', 13" of a stent in accordance with the present invention. Figure 3a is a cross-section of the section 13 shown in Figure 2a after profiling, and Figure 3b a cross-section of the section 13' shown in Figure 2a after profiling. The profiling process results in the top and bottom portions being substantially flat and/or with the surfaces of the top and bottom portions exhibiting substantially similar absolute radii of curvature.

As shown, the profiling process has moved material from the neutral axis of the support member, the side portions 24a and 26a, to the more stressed regions of the stent, the top and bottom portions 20a and 22a, for the stent in the circumferential plane. In addition, the cross-section of a profiled support member has a smaller lumen wall stand-off thickness, which increases the overall inner diameter of the stent thereby increasing lumen size.

Figure 4 is an isometric view of a Boneau stent similar to that shown in Figure 1 which has undergone the process of swaging in accordance with the present invention to produce a profiled stent 30. As shown, each of the sections 32 comprising the stent 30 have two opposing flat sides and rounded edges. The effect of profiling the support members of a stent can also be seen by comparing an end view of a non-profiled stent with the end view of a profiled stent.

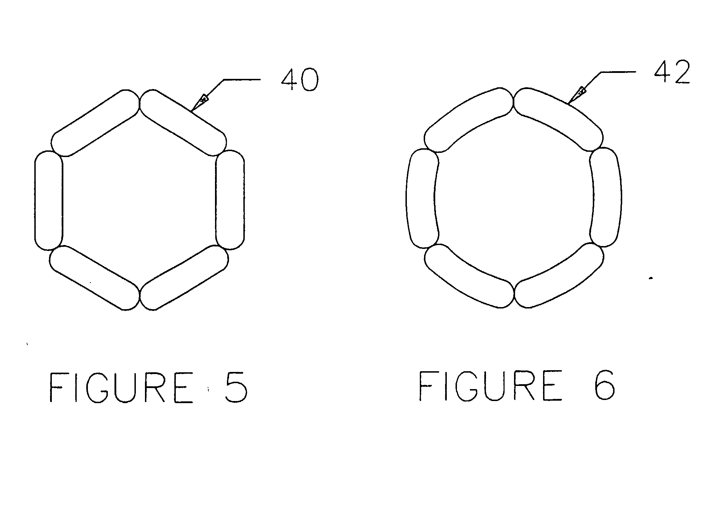

Figure 5 is an end view of a six crown non-profiled Boneau stent. And Figure 6 is an end view of a six crown profiled Boneau stent. Both the non-profiled stent 40 and the profiled stent 42 are shown in compressed form and rolled down onto a catheter (not shown); therefore, only the crowns of the stent are visible. Due to the resulting smaller cross-section shape, the crowns of the profiled stent 42 appear longer and narrower than the crowns of the non-profiled stent 40.

Profiling a stent 42 in this manner has many advantages including increasing the radial strength of the stent, and increasing the efficiency of surface coverage. Furthermore, the rounded edges are retained on the stent, which provides less traumatic trackability as the stent is advanced through a lumen. This avoids dissection of the lumen as might occur with tubular-slotted stents. [In addition, since the profiled stent contains the same volume of material it maintains its radiopacity or visibility during fluoroscopy.]

In a preferred embodiment, the entire stent is profiled. The stent however could be preferentially profiled by profiling only the struts 13 or only the crowns 14 where most of the stress occurs in the instanie of a multi-section stent, or by selectively profiling one or more stent sections. US 5,868,780 entitled, "STENTS FOR SUPPORTING LUMENS IN LIVING TISSUE" discloses a strain relief stent in which the end sections of the stent are circumferentially weaker than the middle sections of the stent. The method of profiling a stent in accordance with the present invention may be used to create such a strain relief stent. This may be done by profiling only the middle sections of the stent, leaving the end sections unprofiled. The profiled sections will have a thicker web, causing the material to plastically deform at a lower deflection, since the rounded cross-sections of the non-profiled end-sections will be more flexible and more resilient than the middle sections.

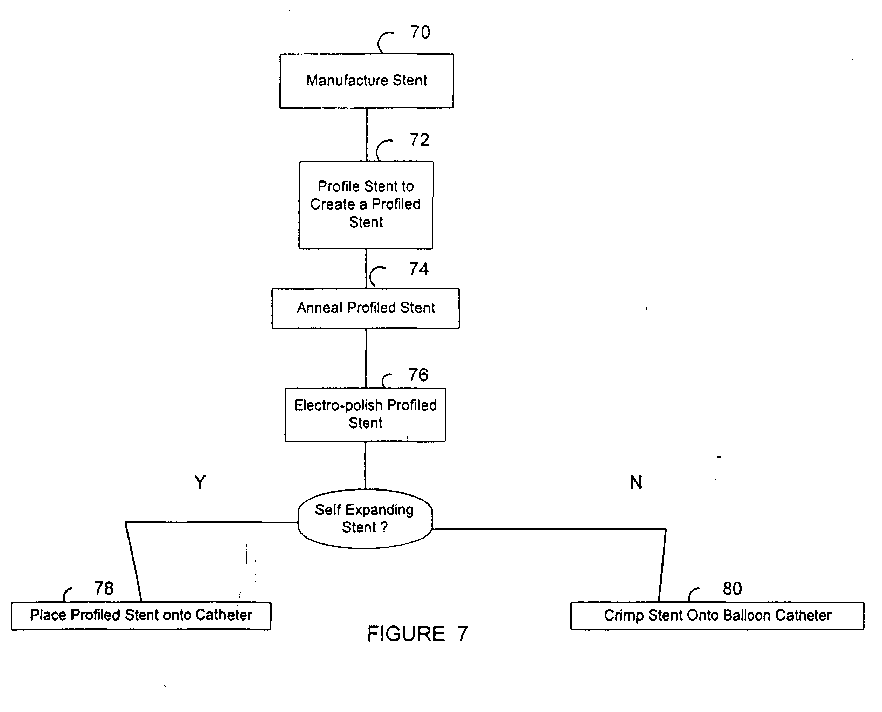

Figure 7 is a flow chart depicting the process of producing a profiled stent in accordance with the present invention. The process begins by manufacturing a conventional stent in step 70. The particular type of stent manufactured may include self-expandable stents or balloon-expandable stents, and tubular-slotted stents or wire-like stents as described above.

After the stent is manufactured, the stent is swaged in order to calibrate the walls of the stent to a desired thickness in step 72. After swaging, the profiled stent is annealed in step 74 to soften and distress the material comprising the stent. After annealing, the stent is electropolished in step 76.

If the stent is self expanding, then the stent can be placed on a catheter is step 78. If the stent is a balloon inflatable stent, then the stent is crimped onto a balloon catheter in step 80 for subsequent insertion into a lumen.

Many methods for swaging a stent are available. In one preferred embodiment of the present invention, a stent is profiled by swaging the stent by either using a swaging machine or by using a collet. In another embodiment, the stent is profiled using a roller method.

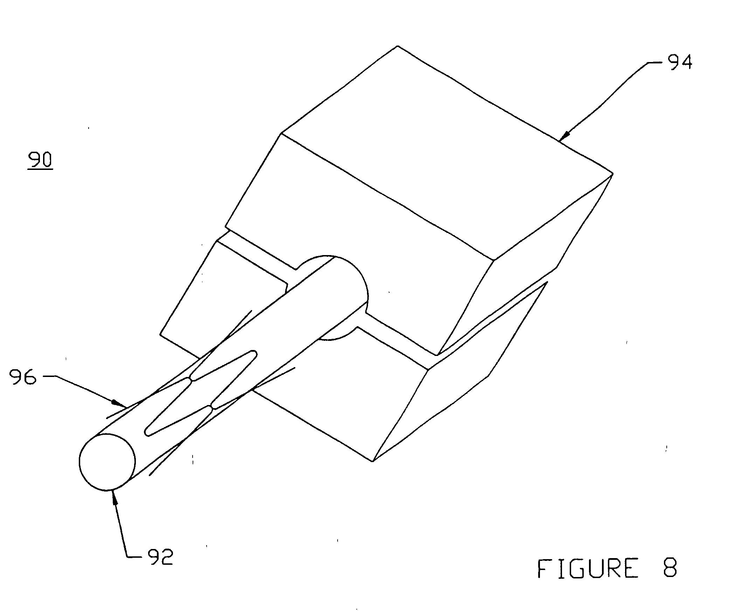

Figure 8 is a block diagram showing the profiling of a stent using a rotary swaging machine 90. The rotary swaging machine 90 includes a mandrel 92 over which a conventional stent is placed, and a die set 94. The stent is swaged by passing the stent and mandrel 92 through the rotating die set 94 while the die set is repeatedly opened and closed. The closed die forces the stent to conform to the annular space defined between the mandrel and the closed die. This plastically deforms the stent. A non-rotary swage machine is also suitable.

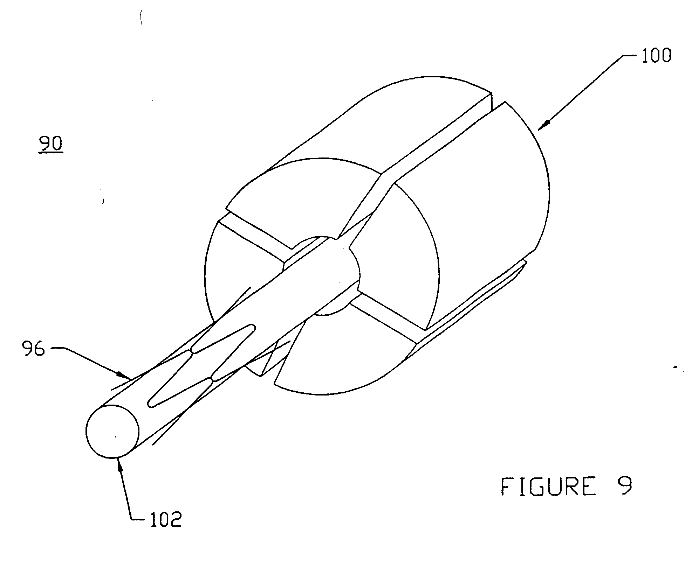

Figure 9 is a block diagram showing the profiling of a stent using a collet 100. Similar to the rotary swage machine 90, a conventional stent is placed over mandrel 102 which is in turn placed into the collet 100. Collet 100 is closed, forcing the stent to conform to the annular space defined between the mandrel 102 and the closed collect 100.

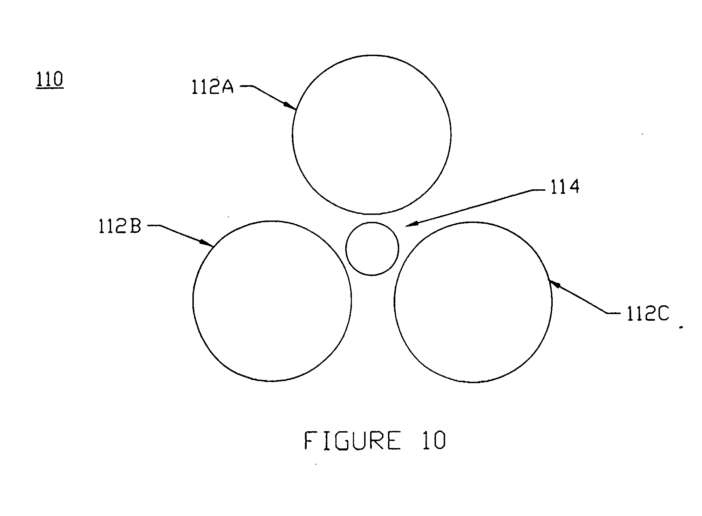

Figure 10 is a block diagram showing the profiling of a stent using a roller machine 110. The roller machine 110 includes a set of three rollers 112a-112c and a mandrel 114 for supporting the stent. Roller 112a is fed into rollers 112b and 112c, thereby compressing the stent against the mandrel 114. The rollers 112 could also be tapered, where the mandrel 114 and the stent are fed through the tapered rollers 112. The thickness of the resulting profiled stent is controlled by the gap between the rollers 112 and the mandrel 114.

A profiled stent and method therefor has been disclosed. Although the present invention has been described in accordance with the embodiments shown, one of ordinary skill in the art will readily recognize that there could be variations to the embodiments and those variations would be within the spirit and scope of the present invention. For example, with respect to tubular-slotted stents, the stent can be cut from a sheet, crushed between a flat plate forming die, and then rolled, with a forming bar or similar tool, and welded. With respect to wire-like stents, after bending the wire into the desired shape it is similarly rolled and the two ends of the wire joined.

Moreover, the instant invention can be used to calibrate the wall thickness of any stent and achieve uniform wall thickness. Additionally, swaging methods of the present invention can be used on any stent material e.g. metal, metal alloy, shape-memory alloy, polymers, etc., that can be plastically deformed. Accordingly, many modifications may be made by one of ordinary skill in the art without departing from the scope of the appended claims.

## Claims

1. A stent comprising a hollow cylindrical body formed of a plurality of elongate support members which extend in a generally axial direction, at least some of the members having a cross section in a plane perpendicular to the axis of the cylinder which has a maximum dimension in the circumferential direction greater than its maximum diameter in the radial direction, **characterised in that** the cross section also has a periphery which is free of corners and has a radially outer face which is substantially flat.

2. The stent according to claim 1 wherein all of the support members have a cross section in a plane perpendicular to the axis of the cylinder which has a maximum dimension in the circumferential direction greater than its maximum diameter in the radial direction, has a smooth periphery and has a radially outer face which is substantially flat.

3. The stent according to claim 1 or claim 2 wherein the cross sectional shape is flattened by a swaging process.

4. The stent according to any preceding claim wherein the stent has a plurality of stent sections.

5. The stent according to claim 1 wherein the radially inner face is substantially flat.

6. A method for producing a stent to be placed in a lumen in living tissue comprising the step of:
manufacturing a stent having a hollow cylindrical body formed of a plurality of elongate support members which extend in a generally axial direction;
**characterised by** compressing at least some of the elongate support members in the radial direction so that their cross section in a plane perpendicular to the axis of the cylinder has a maximum dimension in a circumferential direction greater than its maximum diameter in the radial direction.

7. The method according to claim 6 further including the step of:
thermally processing the profiled stent.

8. The stent according to claim 7 wherein the stent is thermally processed by annealing.

9. The method according to claim 6 further including the step of:
electro-polishing the profiled stent.

10. The method according to claim 6 further including the step of:
profiling the stent using a swage machine.

11. The method according to claim 10 wherein the swage machine includes a mandrel and a die and further includes the steps of:
loading said stent onto the mandrel;
and drawing said mandrel through the die.

12. The method according to claim 6 further including the steps of:
placing the stent into a sleeve;
and advancing a forming tool through said stent in order to compress the cross section of the support members.

## Patentansprüche

1. Stent, der einen hohlen zylindrischen Körper umfasst, der aus einer Vielzahl länglicher Stützelemente besteht, die sich in einer im Allgemeinen axialen Richtung erstrecken, wobei wenigstens einige der Elemente in einer Ebene senkrecht zu der Achse des Zylinders einen Querschnitt haben, der eine maximale Abmessung in der Umfangsrichtung hat, die größer ist als sein maximaler Durchmesser in der radialen Richtung, **dadurch gekennzeichnet, dass** der Querschnitt auch einen Umfang hat, der frei von Ecken ist, und eine radial außen liegende Fläche hat, die im Wesentlichen flach ist.

2. Stent nach Anspruch 1, wobei alle Stützelemente in einer Ebene senkrecht zu der Achse des Zylinders einen Querschnitt haben, der eine maximale Abmessung in der Umfangsrichtung hat, die größer ist als sein maximaler Durchmesser in der radialen Richtungen, einen glatten Umfang hat und eine radial außen liegende Fläche hat, die im Wesentlichen flach ist.

3. Stent nach Anspruch 1 oder Anspruch 2, wobei die Querschnittsform mit einem Senkdrückverfahren abgeflacht wird.

4. Stent nach einem der vorangehenden Ansprüche, wobei der Stent eine Vielzahl von Stent-Segmenten aufweist.

5. Stent nach Anspruch 1, wobei die radial innen liegende Fläche im Wesentlichen flach ist.

6. Verfahren zum Herstellen eines Stents, der in ein Lumen in lebendem Gewebe einzusetzen ist, das die folgenden Schritte umfasst:
Fertigen eines Stents mit einem hohlen zylindrischen Körper, der aus einer Vielzahl länglicher Elemente besteht, die sich in einer im Allgemeinen axialen Richtung erstrecken, **gekennzeichnet durch** das Zusammendrücken wenigstens einiger der länglichen Stützelemente in der radialen Richtung, so dass ihr Querschnitt in einer Ebene senkrecht zu der Achse des Zylinders eine maximale Abmessung in einer Umfangsrichtung hat, die größer ist als sein maximaler Durchmesser in der radialen Richtung.

7. Verfahren nach Anspruch 6, das des Weiteren den folgenden Schritt einschließt:
Wärmebearbeiten des profilierten Stents.

8. Stent nach Anspruch 7, wobei der Stent durch Glühen wärmebearbeitet wird.

9. Verfahren nach Anspruch 6, das des Weiteren den folgenden Schritt einschließt:
Elektropolieren des profilierten Stents.

10. Verfahren nach Anspruch 6, das des Weiteren den folgenden Schritt einschließt:
Profilieren des Stents unter Einsatz einer Gesenkdrückmaschine.

11. Verfahren nach Anspruch 10, wobei die Gesenkdrückmaschine einen Dorn und ein Gesenk enthält, und das des Weiteren die folgenden Schritte einschließt:
Aufsetzen des Stents auf den Dorn;
und Ziehen des Dorns durch das Gesenk.

12. Verfahren nach Anspruch 6, das des Weiteren die folgenden Schritte einschließt:
Einlegen des Stents in eine Buchse;
und Schieben eines Formwerkzeugs durch den Stent, um den Querschnitt der Stützelemente zusammenzudrücken.

## Revendications

1. Un stent comprenant un corps cylindrique creux, formé d'une pluralité d'organes support allongés, s'étendant en direction globalement axiale, au moins certains des organes ayant une section transversale dans un plan perpendiculaire à l'axe du cylindre, ayant une dimension maximale dans la direction circonférentielle supérieure à son diamètre maximal en direction radiale, **caractérisé en ce que** la section transversale a également une périphérie exempte d'angles et a une face radialement extérieure, sensiblement plate.

2. Le stent selon la revendication 1, dans lequel la totalité des organes support ont une section transversale dans un plan perpendiculaire à l'axe du cylindre, ayant une dimension maximale dans la direction circonférentielle supérieure à son diamètre maximal en direction radiale, à une périphérie lisse et une face radialement extérieure, sensiblement plate.

3. Le stent selon la revendication 1 ou la revendication 2, dans lequel la forme de la section transversale est aplatie par un processus de matriçage.

4. Le stent selon l'une quelconque des revendications précédentes, dans lequel le stent présente une pluralité de sections de stent.

5. Le stent selon la revendication 1, dans lequel la face radialement intérieure est sensiblement plate.

6. Un procédé de fabrication d'un stent à placer dans un lumen dans un tissu vivant, comprenant l'étape consistant à :
fabriquer un stent ayant un corps cylindrique creux, formé d'une pluralité d'organes support allongés;
qui s'étendent en direction globalement axiale, **caractérisée par** la compression d'au moins certains des organes support allongés en direction radiale, de manière que leur section transversale, dans un plan perpendiculaire à l'axe du cylindre, soit d'une dimension maximale en direction circonférentielle supérieure à son diamètre maximal en direction radiale.

7. Le procédé selon la revendication 6, comprenant en outre l'étape consistant à :
traiter thermiquement le stent profilé.

8. Le stent selon la revendication 7, dans lequel le traitement thermique du stent est un recuit.

9. Le procédé selon la revendication 6, comprenant en outre l'étape de :
électro-polissage du stent profilé.

10. Le procédé selon la revendication 6, comprenant en outre l'étape consistant à :
profiler le stent en utilisant une estampeuse.

11. Le procédé selon la revendication 10, dans lequel l'estampeuse comprend un mandrin et une filière et comprend en outre les étapes consistant à:
charger ledit stent sur le mandrin;
et étirer ledit mandrin à travers la filière

12. Le procédé selon la revendication 6, comprenant en outre les étapes consistant à :
placer le stent dans une douille;
et faire avancer un outil de formage à travers ledit stent pour comprimer la section transversale des organes support.
